# EUROPEAN PATENT APPLICATION

(11) **EP 4 385 493 A1**
(43) Date of publication of application: **19.06.2024**
(21) Application number: 21953036.7
(22) Date of filing: 12.08.2021
(51) Int. Cl.: A61K 8/97, A61K 8/99, A61Q 19/00, A61Q 19/08

(54) **COSMETIC COMPOSITION, USE OF CASEARIA SYLVESTRIS, SCHINUS TEREBINTHIFOLIUS, HYMENAEA COURBARIL AND LACTOBACILLUS, AND METHOD FOR PREVENTING AND OR TREATING SIGNS OS SKIN AGEING**

(71) Applicant: Natura Cosméticos S.A., 05106-000 São Paulo SP (BR)
(72) Inventor: SOLDATI, Pedro Paulo, 05106-000 São Paulo - SP (BR); ZIMBARDI, Daniela, 05106-000 São Paulo - SP (BR); DURÃES ZERBINATTI, Gabriela, 05106-000 São Paulo - SP (BR); DE MIRANDA CHAVES VASQUEZ PINTO, Luciana, 05106-000 São Paulo - SP (BR); CAROLLO MONCAYO, Priscila, 05106-000 São Paulo - SP (BR); ARANDAS MONTEIRO E SILVA, Silas, 05106-000 São Paulo - SP (BR); PANZARIN SAVIETTO, Joice, 05106-000 São Paulo - SP (BR); ABRAHÃO DIAS, Ana Luisa, 05106-000 São Paulo - SP (BR)
(74) Representative: Simmons & Simmons
(86) International application number: PCT/BR2021/050341
(87) International publication number: WO 2023/015364

(57) **Abstract**

The present invention relates to cosmetic compositions comprising *Casearia sylvestris* (wild sage), *Schinus terebinthifolius* (Brazilian pepper tree), *Hymenaea courbaril* (Brazilian copal), *Lactobacillus* and cosmetically acceptable adjuvants, in diverse cosmetic forms, which are useful in preventing and/or treating signs resulting from skin aging. The present invention further relates to the use of *Casearia sylvestris, Schinus terebinthifolius, Hymenaea courbaril* and *Lactobacillus,* in combination, for the prevention and/or treatment of signs resulting from skin aging, as well as a method for the prevention and/or treatment of signs resulting from skin aging.

## Description

### FIELD OF THE INVENTION

The present invention relates to cosmetic compositions comprising *Casearia sylvestris* (wild sage), *Schinus terebinthifolius* (Brazilian pepper tree), *Hymenaea courbaril* (Brazilian copal), *Lactobacillus* and cosmetically acceptable adjuvants, in diverse cosmetic forms, which are useful in preventing and/or treating signs resulting from skin aging. The present invention further relates to the use of *Casearia sylvestris, Schinus terebinthifolius, Hymenaea courbaril* and *Lactobacillus,* in combination, for the prevention and/or treatment of signs resulting from skin aging, as well as a method for the prevention and/or treatment of signs resulting from skin aging.

### BACKGROUND OF THE INVENTION

The skin covers the surface of the human body and is made up of an epithelial portion, the epidermis, and a connective portion, the dermis. The skin is one of the largest organs in the human body and performs multiple functions such as protecting the body against water loss and friction, transmitting information about the environment to the central nervous system, contributing to the body's thermoregulation and excretion of various substances, among others.

The epidermis is made up of keratinized stratified squamous epithelium. The dermis, in turn, is made up of connective tissue that supports the epidermis and joins the skin to the subcutaneous tissue.

Skin care, in all its extensions, has been the object of numerous investigations in recent years, especially for aesthetic reasons, associated with the prevention and/or treatment of signs resulting from skin aging.

Several mechanisms are involved in skin aging. Among them is the decreased production of collagen, a protein responsible for the structure and firmness of the skin which faces a progressive decrease after a certain age due to the aging of fibroblasts, causing reduced production of fibers. Other important components of the skin structure, such as elastin, hyaluronic acid and proteins that join the skin layers such as integrins, also have their production progressively reduced with age, affecting the structure and functions of the skin.

Thus, over the years, the market has made available numerous alternative cosmetic compositions aimed at preventing and/or treating signs resulting from skin aging, particularly with regard to intervention in the production or supplementation of collagen, as this is responsible by skin strength.

However, there remains a need for new products that are effective in preventing and/or treating signs resulting from skin aging, particularly that act more efficiently in stimulating the production of collagen and other structural proteins such as elastin and integrins, in addition to substances relevant to the skin such as hyaluronic acid, among other mechanisms and benefits.

### SUMMARY OF THE INVENTION

In a first embodiment, the present invention relates to a cosmetic composition comprising *Casearia sylvestris* (wild sage), *Schinus terebinthifolius* (Brazilian pepper tree), *Hymenaea courbaril* (Brazilian copal), *Lactobacillus* and cosmetically acceptable adjuvants, in appropriate amounts to provide unexpected effects in the prevention and/or treatment of signs resulting from skin aging.

In another embodiment, the present invention relates to the use of *Casearia sylvestris* (wild sage), *Schinus terebinthifolius* (Brazilian pepper tree), *Hymenaea courbaril* (Brazilian copal) and *Lactobacillus* in the preparation of a cosmetic composition efficient in preventing and/or treating signs resulting from skin aging.

In a third embodiment, the present invention refers to the method of preventing and/or treating signs resulting from skin aging comprising applying to the skin a cosmetic composition according to the present invention.

### BRIEF DESCRIPTION OF THE FIGURES

Figure 1 shows the fluorescence intensity semi-quantification of the synthesis of type I collagen in biopsies obtained from volunteers treated with the composition of the present invention. Data represent the mean ± standard deviation of 9 areas (ANOVA - Bonferroni).
Figure 2 shows the fluorescence intensity semi-quantification of the synthesis of elastin in biopsies obtained from volunteers treated with the composition of the present invention. Data represent the mean ± standard deviation of 9 areas (ANOVA - Bonferroni).
Figure 3 shows the fluorescence intensity semi-quantification of the synthesis of hyaluronic acid in biopsies obtained from volunteers treated with the composition of the present invention. Data represent the mean ± standard deviation of 9 areas (ANOVA - Bonferroni).
Figure 4 shows the fluorescence intensity semi-quantification of the synthesis of integrins in biopsies obtained from volunteers treated with the composition of the present invention. Data represent the mean ± standard deviation of 9 areas (ANOVA - Bonferroni).
Figure 5 shows the CFU after 24h of incubation in MCM + test ingredient.

### DESCRIPTION OF THE INVENTION

The present invention relates to a cosmetic composition effective in preventing and/or treating signs resulting from skin aging.

The applicant has surprisingly found that the cosmetic composition according to the present invention, which comprises *Casearia sylvestris* (wild sage), *Schinus terebinthifolius* (Brazilian pepper tree), *Hymenaea courbaril* (Brazilian copal), *Lactobacillus* and cosmetically acceptable adjuvants, exhibits unexpected results, that is, synergistic results in the production of collagen, in addition to simultaneous beneficial effects on the skin, such as increased luminosity, improved texture, increased elastin production, immediate hydration and hydration for up to 48 hours.

According to the present invention, *Casearia sylvestris* (wild sage) and *Schinus terebinthifolius* (Brazilian pepper tree) can be used in the form of an extract and *Hymenaea courbaril* (Brazilian copal) in the form of an extract, oil or butter. In a particular embodiment, said ingredients are used in extract form. In a preferred embodiment, Lactobacillus is selected from *Lactobacillus plantarum.*

In a preferred embodiment, the cosmetic composition according to the present invention comprises from 0.001 to 5% *Casearia sylvestris,* from 0.001 to 5% *Schinus terebinthifolius,* from 0.001 to 5% *Hymenaea courbaril* and from 0.001 to 2% *Lactobacillus* by weight of the composition.

In an even more preferred embodiment, the cosmetic composition according to the present invention comprises 0.1% *Casearia sylvestris,* 0.025% *Schinus terebinthifolius,* 0.25% *Hymenaea courbaril* and 0.5% *Lactobacillus* by weight of the composition.

Simultaneous results on the skin provided by the composition of the present invention are immediately seen in terms of luminosity, smoothing of fine lines, improvement in texture, immediate hydration and hydration for up to 48 hours. After seven days there is a visible reduction in fine lines (mainly located in the area around the eyes). After 15 days, there is noted a significant reduction in the number and depth of moderate wrinkles located mainly in the so-called Chinese mustache and, surprisingly, there is up to 75% more collagen, elastin and hyaluronic acid, in addition to a strengthened skin microbiota due to prebiotic and probiotic effects. Furthermore, after 30 days, there is noted a restructured skin, improved skin density and reorganization of layers, the presence of twice as many proteins in the skin connection and an intensive reduction of deep wrinkles mainly located in the forehead region.

The cosmetic composition according to the present invention may be available in different cosmetically acceptable forms, for example, but not limited to, in the form of a cream, gel, suspension, emulsion, elixir, serum, oil, ointment.

The cosmetic composition according to the present invention is intended for use in the prevention and/or treatment of signs resulting from skin aging, with effects on the skin microbiota (prebiotic and probiotic effects), moisturizing effects (luminous skin), increased collagen production, increased elastin production, increased hyaluronic acid production, increased integrin production, wrinkle reduction and redensification of the dermis.

Thus, in a second aspect, the present invention relates to the use of *Casearia sylvestris, Schinus terebinthifolius, Hymenaea courbaril* and *Lactobacillus* in the preparation of a cosmetic composition according to the present invention for preventing and/or treating signs resulting from skin aging.

Also, in a third aspect, the present invention relates to the method of preventing and/or treating signs resulting from skin aging comprising applying to the skin a cosmetic composition according to the present invention.

Cosmetically acceptable adjuvants according to the present invention include, without limitation, those known in the art. In particular embodiments, the ingredients may include emollients, humectants, emulsifiers, conditioning agents, sensory modifiers, silicones, oils, rheological agents, opacifiers, thickeners, structuring agents, antioxidants, chelants, neutralizers, pigments and sensory particles, fragrances, preservatives, solubilizers, glycols, among others. More particularly, the ingredients may be selected from water, acrylates, pentylene glycol, ethoxylates, gums, glycerin, propanediol, silicas, dimethicone, fatty acids, fatty alcohols, caprylic/capric triglyceride, ethyl macadamiate, arachidyl alcohol, behenyl alcohol and arachidyl glucoside, trehalose, jojoba esters, dicaprylyl carbonate, phenoxyethanol, hydroxyacetophenone, perfume, xanthan gum, cetearyl alcohol, sodium gluconate and/or tocopherol.

The following examples, without imposing any limitation, illustrate the present invention, particularly with regard to the effects on the quantification of molecular markers associated with general skin functions.

### EXAMPLES

### Example 1. Cosmetic composition according to the present invention

The cosmetic composition according to the present invention was prepared using 0.1% wild sage extract (*Casearia sylvestris*)*,* 0.025% Brazilian pepper tree extract (*Schinus terebinthifolius*)*,* 0.25% Brazilian copal extract (*Hymenaea courbaril*) and 0.5% *Lactobacillus* by weight of the composition.

The adjuvants used were water, glycerin, propanediol, silica, dimethicone, caprylic/capric triglyceride, ethyl macadamiate, arachidyl alcohol, behenyl alcohol and arachidyl glucoside, trehalose, jojoba esters, dicaprylyl carbonate, phenoxyethanol, hydroxyacetophenone, perfume, xanthan gum, cetearyl alcohol, sodium gluconate and tocopherol.

### Example 2. Assessment of the synthesis of epidermal and dermal proteins

The objective of the study was to evaluate the clinical effects of the composition of the present invention on the synthesis of type I collagen, elastin, hyaluronic acid, α6β4 integrin in human skin biopsies by immunofluorescence analysis, to demonstrate anti-aging and filler effects.

Nine volunteers completed the study within 28 days after use of the test product; average age: 48 ± 05 years old. There were no reports or evidence of side reactions during the study.

Research volunteers were instructed to stop using any cosmetic products on their forearms 72 hours prior to the start of the study. On the day of the study, a site was marked on the right and left forearms of the research volunteers and the composition of the present invention was applied to one of the sites and the other site was used as control (contralateral forearm). Human skin biopsies were collected before starting the use of the test product (T0), after 14 days of home use (T14) and after 28 days of home use (T28), followed by immunofluorescence analysis.

The results demonstrated that the composition of the present invention promotes an increased synthesis of type I collagen, elastin, hyaluronic acid, α6β4 integrin, favoring tissue support, elasticity and filling, reducing the effects of aging, such as the appearance of wrinkles and sagging.

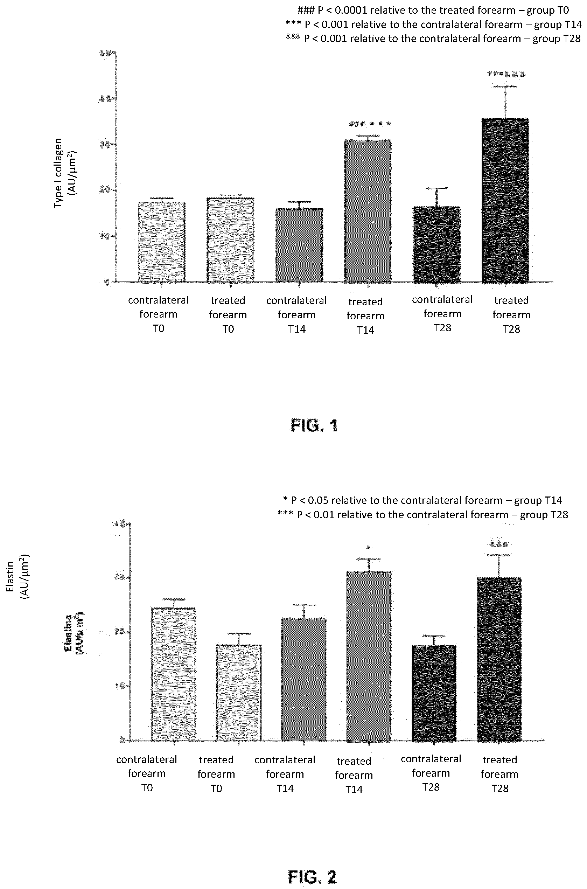

In particular in Figure 1, the staining intensity for type I collagen is significantly increased (69.17%) in the T14 Treated Forearm (composition according to the present invention); (P <0.0001).

Specifically in Figure 2, the staining intensity for elastin is significantly increased by 76.56% in the T14 Treated Forearm (composition according to the present invention); (P <0.01).

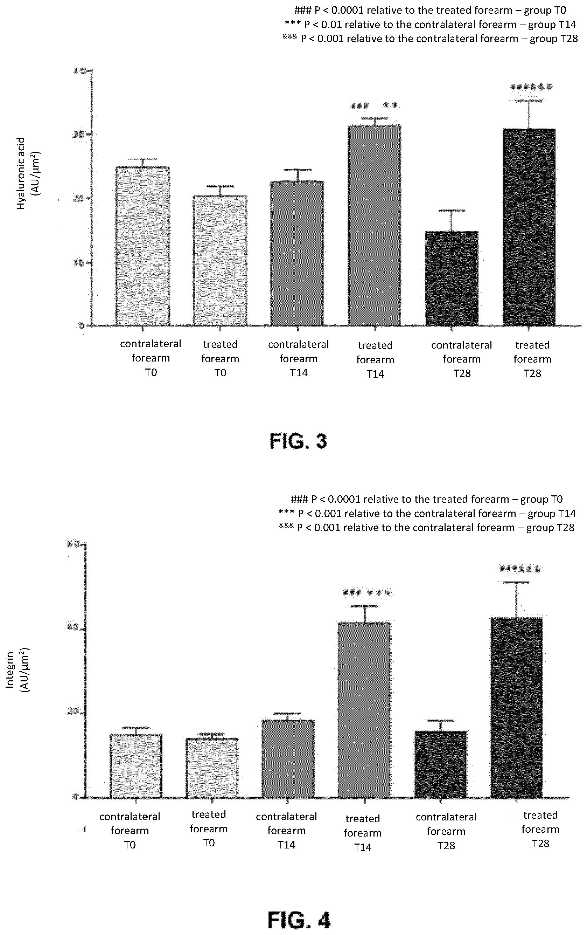

Specifically in Figure 3, the staining intensity for hyaluronic acid is significantly increased by 54.47% in the T14 Treated Forearm (composition according to the present invention); (P <0.01).

Specifically in Figure 4, the staining intensity for integrin is significantly increased by 198.46 the T14 Treated Forearm (composition according to the present invention); (P <0.0001).

The results demonstrated that the composition of the present invention promotes an increased synthesis of type I collagen, elastin, hyaluronic acid, α6β4 integrin, favoring tissue support, elasticity and filling, reducing the effects of aging, such as the in accordance with the wrinkles and sagging.

### Example 3. In vitro assessment of prebiotic activity

Two groups were tested, the first one (Group 1) receiving 0.5% of the composition of the present invention and the second one (Group 2) 0.025% + 0.1% of the composition of the present invention.

The goal of the study was to assess the prebiotic activity considering bacteria from the skin microbiome *in vitro.*

The microorganisms studied were *Staphylococcus warneri,* 3 different strains of *Staphylococcus epidermidis, Staphylococcus aureus, Corynebacterium striatum, Corynebacterium xerosis* and *Cutibacterium acnes.*

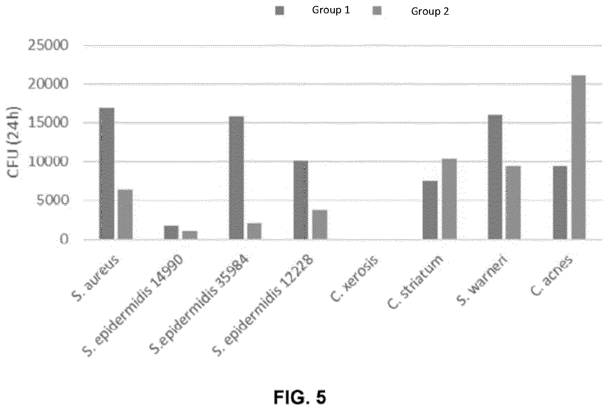

The compositions of the present invention could be metabolized by bacteria and induce growth after 24h of incubation, favoring the beneficial ones, as shown in figure 5. Furthermore, as shown in Table 2, biofilm formation was greater in beneficial microorganisms than in non-beneficial microorganisms.

### Example 4. Assessment of skin roughness by image analysis

Ten research volunteers completed the study; the average ages were: 49 ± 8 years; Phototype (Fitzpatrick): 50% phototype III, 50% phototype IV.

Research volunteers were instructed to stop using any cosmetic products on face and their forearms 48 hours prior to the start of the study.

The methodology consisted of assessing the reduction in dryness and improvement in skin texture through image analysis. Images of the forehead and forearms were obtained at the beginning of the study, 15 minutes and 2 hours after application of the investigational product.

According to the results achieved after applying the protocol, it can be concluded that:
Forearms: there was a significant 12.6% reduction in skin roughness after 15 minutes of the application of the composition of the present invention and there was a significant 7.4% reduction in skin roughness after 2 hours of the application of the composition of the present invention.
Forehead: there was a significant 10.8% reduction in skin roughness after 15 minutes of the application of the composition of the present invention and there was a significant 3.9% reduction in skin roughness after 2 hours of hours of the application of the experimental product.

This is an indication that application of the composition of the present invention provides an improved skin texture.

### Example 5. Assessment of clinical efficacy

Clinical efficacy of the composition of the present invention having dermal redensification properties was assessed by analyzing skin images using 50 MHz ultrasound in a 50 MHz Dub^{®} SkinScanner ultrasound.

To measure the parameters, a 50 MHz high-frequency ultrasound equipment was used, with the aid of a software. Readings were taken by applying the probe to the test area, resting it on the skin on previously demarcated regions. A gel was also used at the interface between the probe and the skin to obtain a better quality image.

The study volunteers consisted of 22 women with ages ranging from 35 to 60 years old. Based on the age distribution of the participants, the majority are between 46 and 55 years of age.

Based on the data obtained, it can be observed that after 60 days of use of the test formulation, there was a significant increase of 36.76%, 18.13% and 21.61% in dermal echo-density in the malar, periorbital and frontal regions, respectively. It can also be noted that in these areas the data distribution tended towards higher values of this parameter at T60 when compared to T0. Therefore, dermis density was suggested to be increased in the studied regions under the influence of the test product.

The composition of the present invention was found to provide a significant increase in dermal echo-density in all facial regions evaluated. The product also caused a significant reduction in SENEB in the malar and frontal sites, with an increase in hyperechoic particles. Furthermore, the dermal thickness of the malar region was significantly reduced due to the horizontal and more compact organization of collagen bundles. Therefore, the use of the product was shown to promote dermal redensification.

### Example 6. Assessment of skin hydration by corneometry.

The object of this study was to assess an improvement in skin hydration after applying the composition of the present invention.

10 research volunteers completed the study. Average age: 46 ± 12 years old.

Research volunteers were instructed to stop using any cosmetic products on their forearms 48 hours prior to the start of the study. Before starting the measurements, the volunteers remained for 30 minutes in an air-conditioned environment at 20 ± 2 °C and 50 ± 5% relative humidity. After the stabilization period, baseline capacitance measurements were obtained. Then, the composition of the present invention was applied and the research volunteers remained in the laboratory for measurements after 15 minutes, 2, 4 and 8 hours. In two following days, research volunteers returned to the laboratory for 24- and 48-hour measurements after applying the product under investigation.

According to the study protocol and the procedures used to assess the increase in skin hydration, the composition of the present invention was shown to provide a significant increase in skin hydration after 15 minutes, 2, 4, 8, 24 and 48 hours of application as compared to control (skin with no products applied).

The composition of the present invention keeps the skin hydrated for up to 48 hours after application.

The composition of the present invention increased the skin hydration level by up to 37.3%.

100% of the research volunteers showed an improvement in skin hydration after applying the composition of the present invention.

### Example 7. In vitro assessment of the potential activity on the synthesis of total collagen and elastin

In this study, the effects of the composition of the present invention on the synthesis of Total Collagen and Elastin in human fibroblast cultures were evaluated in order to assess the potential attenuating activity on the aspects and signs of skin aging.

Normal human dermal fibroblasts (*Gibco, Thermo Scientific*) were seeded in 75 cm² bottles, cultured and expanded in a humid oven at 37 °C in the presence of 5% CO₂, using a specific culture medium. Upon reaching 70-80% confluence, cells were trypsinized and seeded in 6-well plates for subsequent incubation with the test product and evaluation of the proposed parameter.

The cells were incubated with a concentration of each of the test products under reducing conditions of Fetal Bovine Serum to 1%. Then, the cells were incubated under the same culture conditions as above for another 72 hours. Thereafter, the cell supernatant was collected for subsequent measurement of the synthesis of Total Collagen and the cell lysate was collected for quantification of the synthesis of elastin.

Soluble collagen was measured using a commercially available kit (*SIRCOL Soluble Collagen Assay, Biocolor Co., UK*)*.* In plastic microcentrifuge tubes, 1mL of the specific dye is added (*Sircol Dye Reagent*) in 100 µl of the test sample supernatant. Then, the microtubes are kept under agitation in an orbital mixer for 30 minutes at room temperature (RT). The collagen-dye complex formed is precipitated by centrifugation (10,000 x g) for 10 minutes, then the supernatant is discarded and the formed pellet (collagen + dye complex) is suspended in 1mL of an alkali reagent (0.5 M NaOH solution). Then, 200 µL aliquots are transferred to a 96-well plate and the absorbance reading is determined at 540 nm. Collagen concentration is determined based on the calibration curve using a total collagen standard provided by the kit manufacturer.

Elastin levels are determined according to the protocol described in the Fastin detection kit (*Biocolor, Belfast, Ireland*)*.* The elastin content of the samples is precipitated in microcentrifuge tubes after the addition of 1 mL of precipitation reagent (trichloroacetic acid and arginine) and incubation at 0 °C for 24 hours. After centrifuging the tubes (10,000 x g) for 10 minutes, the supernatant is discarded and the pellet is suspended in 1 mL of TPPS (5,10,15,20-tetraphenyl-21,23-porphine sulfonate) and 200 µL of 90% saturated ammonium sulfate for formation of the elastin-dye complex. After 60 minutes of stirring, the tubes are centrifuged (10,000 x g) for 10 minutes, the supernatant is discarded and the formed complex is suspended in 1 mL of dissociation reagent (guanidine HCl and 1-propanol) which allows the formation of a colored complex, the absorbance of which is measured at 513 nm. Elastin concentration is calculated based on the calibration curve using the elastin standard provided by the kit manufacturer.

Analysis of variance (ANOVA) was used for statistical analysis. Dunnett's test was used when the analysis of variance detected significant differences between the groups. In all groups studied, those with P values of less than 0.05 were considered statistically significant.

### Different samples were tested:

Sample 1 represents 0.01% wild sage + 0.0025% Brazilian pepper tree + 0.1% *bifidobacterium*;
Sample 2 represents 0.01% wild sage + 0.0025% Brazilian pepper tree + 0.25% Brazilian copal + 0.1% *bifidobacterium*;
Sample 3 represents 0.01% wild sage + 0.0025% Brazilian pepper tree + 0.25% Brazilian copal + 0.1% sodium cocoyl amino acids (for example, available under the trade name Sepicalm) + 0.1% *Bifidobacterium*;
Sample 4 represents 0.01% wild sage + 0.0025% Brazilian pepper tree + 0.1% *Lactobacillus*;
Sample 5 represents 0.01% wild sage + 0.0025% Brazilian pepper tree + 0.25% Brazilian copal + 0.1% *Lactobacillus*;
Sample 6 represents 0.01% wild sage + 0.0025% Brazilian pepper tree + 0.25% Brazilian copal + 0.1% sodium cocoyl amino acids (for example, available under the trade name Sepicalm) + 0.1% *Lactobacillus*;
Sample 7 represents 0.1% *Bifidobacterium*;
Sample 8 represents 0.1% *Lactobacillus.*

All samples promoted a significant increase (P<0.01) in Total Collagen synthesis *in vitro,* by 411, 1237, 370, 701, 2636, 388, 870 and 1417%, respectively, compared to the Control group.

However, unexpectedly and surprisingly, based on the above test results as well as additional studies carried out under the same test conditions, a synergistic effect of the composition according to the present invention was observed on total collagen (commercially available kit *SIRCOL Soluble Collagen Assay, Biocolor Co.*)*,* according to the following table:

**Table 1. Results with total collagen marker; basaline control**

| Sample | Concentrations | Results |
|---|---|---|
| Wild sage + Brazilian pepper tree + *Lactobacillus* | 0.1% + 0.025% + 0.5% | 701% |
| Brazilian copal | 0.25 % (w/v) | 268% |
| Brazilian copal | 0.125 % (w/v) | 249% |
| Brazilian copal | 0.060 % (w/v) | 44% |
| Wild sage + Brazilian pepper tree + Brazilian copal + *Lactobacillus* | 0.1% + 0.025% + 0.25% + 0.5% | 2636% |

The results show that the composition according to the present invention exhibited a synergistic result of 2636% in the total collagen as compared to the results of 701% for wild sage + Brazilian pepper tree + *Lactobacillus* and 268% for Brazilian copal.

The results presented in the present study also demonstrate that the composition according to the present invention can promote a significant increase (P<0.01) in the synthesis of elastin of 15%.

Such results demonstrate the skin anti-aging effect of the composition according to the present invention.

The person skilled in the art will be able to readily assess through the teachings of the instant text and examples the advantages of the invention and to propose variations and equivalent alternatives of implementation without departing from the scope of the invention as defined in the appended claims.

## Claims

1. A cosmetic composition **characterized in that** it comprises *Casearia sylvestris, Schinus terebinthifolius, Hymenaea courbaril, Lactobacillus* and cosmetically acceptable adjuvants.

2. The cosmetic composition according to claim 1, **characterized in that** it comprises from 0.001 to 5% *Casearia sylvestris,* from 0.001 to 5% *Schinus terebinthifolius,* from 0.001 to 5% *Hymenaea courbaril* and from 0.001 to 2% *Lactobacillus* by weight of the composition.

3. The cosmetic composition according to claim 1, **characterized in that** it comprises from 0.001 to 0.2% *Casearia sylvestris,* from 0.001 to 1% *Schinus terebinthifolius,* from 0.001 to 1% *Hymenaea courbaril* and from 0.001 to 1% *Lactobacillus* by weight of the composition.

4. The cosmetic composition according to claim 2, **characterized in that** it comprises 0.1% *Casearia sylvestris,* 0.025 % Schinus terebinthifolius, 0.25% *Hymenaea courbaril* and 0.5% *Lactobacillus* by weight of the composition.

5. The cosmetic composition according to any one of claims 1 to 3, **characterized in that** it is in the form of a cream, gel, suspension, emulsion, elixir, serum, oil, ointment.

6. The cosmetic composition according to any one of claims 1 to 4, **characterized in that** *Casearia sylvestris* and *Schinus terebinthifolius are* in form of an extract and *Hymenaea courbaril* in the form of extract, oil or butter.

7. The cosmetic composition according to any one of claims 1 to 4, **characterized in that** the *Lactobacillus* is selected from *Lactobacillus plantarum.*

8. The cosmetic composition according to any one of claims 1 to 7, **characterized in that** it is for use in preventing and/or treating signs resulting from skin aging.

9. Use of *Casearia sylvestris, Schinus terebinthifolius, Hymenaea courbaril* and *Lactobacillus,* **characterized in that** it is in the preparation of a cosmetic composition as defined in any one of claims 1 to 8, for preventing and/or treating signs resulting from skin aging.

10. A method of preventing and/or treating signs resulting from skin aging, **characterized in that** it comprises applying on the skin a cosmetic composition as defined in any one of claims 1 to 8.
